# EUROPÄISCHE PATENTSCHRIFT

(11) **EP 1 505 156 B1**
(45) Veröffentlichungstag und Bekanntmachung des Hinweises auf die Patenterteilung: **07.12.2005**
(21) Anmeldenummer: 04017997.0
(22) Anmeldetag: 29.07.2004
(51) Int. Cl.: C12P 41/00, C12P 7/04, C12P 7/20

(54) **Verfahren zur enantioselektiven Herstellung von sekundären Alkoholen durch lipasenkatalysierte Solvolyse der korrespondierenden Acetessigester**
Process for the enantioselective preparation of secondary alcohols by lipase catalysed solvolysis of the corresponding acetoacetic acid ester
Procédé de préparation énantiosélective d'alcools secondaires d'un ester de l'acide acéto-acétique obtenus par solvolyse par une lipase

(30) Priorität: 07.08.2003 DE 10336270
(43) Veröffentlichungstag der Anmeldung: 09.02.2005
(73) Patentinhaber: Consortium für elektrochemische Industrie GmbH, 81379 München (DE)
(72) Erfinder: Popp, Alfred, Dr., 82008 Unterhaching (DE); Stohrer, Jürgen, Dr., 82049 Pullach (DE); Petersen, Hermann, Dr., 84489 Burghausen (DE); Gilch, Andrea, 85419 Mauern (DE)
(74) Vertreter: Potten, Holger

(56) Entgegenhaltungen:
- EP-A- 0 321 918
- EP-A- 0 556 909
- WO-A-95/06746
- US-A- 5 604 120
- US-A1- 2002 102 671
- LALLEMAND J-Y ET AL: "Easy access to an optically pure precursor of forskolin" TETRAHEDRON ASYMMETRY 1993 UNITED KINGDOM, Bd. 4, Nr. 8, 1993, Seiten 1775-1778, XP002294663 ISSN: 0957-4166
- C RDOVA A ET AL: "A highly chemo- and stereoselective synthesis of [beta]-keto esters via a polymer-supported lipase catalyzed transesterfication" JOURNAL OF ORGANIC CHEMISTRY 09 MAR 2001 UNITED STATES, Bd. 66, Nr. 5, 9. März 2001 (2001-03-09), Seiten 1906-1909, XP002294664 ISSN: 0022-3263
- JEROMIN G E ET AL: "Diketene a New Esterification Reagent in the Enzyme-Aided Synthesis of Chiral Alcohols and Chiral Acetoacetic Acid Esters" TETRAHEDRON LETTERS, ELSEVIER SCIENCE PUBLISHERS, AMSTERDAM, NL, Bd. 36, Nr. 37, 11. September 1995 (1995-09-11), Seiten 6663-6664, XP004027308 ISSN: 0040-4039
- SUGINAKA K ET AL: "Highly Selective Resolution of Secondary Alcohols and Acetoacetates with Lipases and Diketene in Organic Media" TETRAHEDRON: ASYMMETRY, ELSEVIER SCIENCE PUBLISHERS, AMSTERDAM, NL, Bd. 7, Nr. 4, 1. April 1996 (1996-04-01), Seiten 1153-1158, XP004047786 ISSN: 0957-4166

## Beschreibung

Die Erfindung betrifft ein enantioselektives Verfahren zur Herstellung sekundären Alkoholen durch enantioselektive lipasenkatalysierte Solvolyse der korrespondierenden Acetessigester.

Immer mehr enantiomerenreine Verbindungen dienen als Ausgangsmaterialien bzw. Zwischenprodukte bei der Synthese von Agrochemikalien und Pharmazeutika. Bislang werden und wurden jedoch viele dieser Verbindungen als Racemat oder Diastereomerengemisch hergestellt und vermarktet. In vielen Fällen wird der gewünschte physiologische Effekt aber nur von einem Enantiomer bzw. Diastereomer bewirkt. Das andere Isomer ist im günstigsten Fall inaktiv, es kann aber auch dem gewünschten Effekt entgegenwirken oder sogar toxisch sein. Verfahren zur Trennung von Racematen werden deshalb immer wichtiger für die Darstellung enantiomerenreiner oder hochenantiomerenangereicherter Verbindungen.

Aus dem Stand der Technik ist bekannt, dass die Racemattrennung chiraler Verbindungen mit Hilfe von Enzymen durchgeführt werden kann. In einer Vielzahl von Publikationen wird als mögliche Methode die enzymatisch kinetische Racematspaltung von Estern mit Lipasen und Esterasen beschrieben. Die Racematspaltung von optisch aktiven sekundären Alkoholen wird im Allgemeinen durch Acylierung der Hydroxylgruppe am stereogenen Zentrum oder umgekehrt durch Hydrolyse des korrespondierenden Esters durchgeführt.

Janda et al. beschreiben in US 2002/0102671 A1 und J. Org. Chem. 2001, 66, S. 1906-1909 die direkte lipasenkatalysierte Racematspaltung sekundärer Alkohole durch Umesterung mit β-Keto-Estern. Nachteil dieser Methode ist, dass die als Acylierungsmittel verwendeten β-Keto-Ester zunächst in einer separaten Synthese (im allgemeinen) aus Diketen hergestellt werden müssen, wodurch zusätzliche Umarbeitungskosten entstehen.

Für die Acylierung werden vor allem Carbonsäureester, bei denen eine Rückreaktion des gebildeten Esters durch eine nachgelagerte Reaktion verhindert wird (Gleichgewichtsreaktion bzw. Gleichgewichtsverschiebung durch Umesterung), verwendet.

EP 321918 B2 beschreibt dabei die Umesterung eines Vinylesters durch enzymatisch bedingte enantioselektive Reaktionsführung. Bei der Umesterung entstehen dann aus den intermediär freigesetzten Vinylalkoholen irreversibel Aldehyde oder Ketone (z. B. Acetaldehyd oder Aceton), die jedoch aus der Reaktionsmischung aufwändig abgetrennt werden müssen, da sie die eingesetzten Enzyme inaktivieren können.

Dieser Nachteil wird durch die Verwendung von Diketen als Acylierungsmittel unter Bildung der entsprechenden Acetessigester vermieden, da in diesem Fall keine Spaltungsprodukte bei der Acylierung entstehen. Die Verwendung von Diketen als Acylierungsmittel ist aus dem Stand der Technik ebenfalls bekannt.

In EP 716712 B1 wird ein Verfahren zur Herstellung von enantioselektiv acylierten Alkoholen durch Umsetzung eines racemischen Alkohols mit einem Diketen unter spezifischer Lipasenkatalyse beschrieben. Dabei werden jedoch nur niedrige Enantioselektivitäten erreicht. So erhält man durch die enzymkatalysierte Umsetzung von (*rac*)-1-Phenylethanol mit Diketen lediglich eine Enantioselektivität *E* von 28, wobei *E* gemäß der Definition von Chen, C. et al. in *J. Am. Chem. Soc.* 1982, 104, S. 7294-7299 zu verstehen ist.

Auch Jeromin, G. et al. in *Tetrahedron Lett.* 1995, 36(37), S. 6663-6664 und Suginaka, K. et al. in *Tetrahedron: Asymmetry* 1996, 7(4), S. 1153-1158 haben weitere Verfahren zur Herstellung von enantioselektiv acylierten Alkoholen durch Umsetzung eines racemischen Alkohols mit einem Diketen beschrieben. Auch nach diesen Verfahren sind lediglich geringe Enantioselektivitäten (E meist wesentlich kleiner als 50) zu erreichen. Ferner werden nur niedrige Enantiomerenreinheiten und geringe chemische Ausbeuten erzielt.

Bekannt ist auch die, durch Esterasen (Klasse 3.1.1.1 gemäß Internationaler Enzym-Nomenklatur, Committee of the International Union of Biochemistry and Molecular Biology) katalysierte Hydrolyse von Acetessigestern (Lallemand, J.-Y. et al. in *Tetrahedron: Asymmetry*. 1993, 4(8), S. 1775-1778). Darin wird die mit Schweineleberesterase (PLE) katalysierte Umsetzung eines Acetoacetats mit Wasser zum entsprechenden Alkohol beschrieben. Die Verwendung von Esterasen bringt zum einen jedoch den erheblichen Nachteil, dass diese (z. B. Pferd- oder Schweineleberesterase) zumeist tierischen Ursprungs sind und sich deren Einsatz bei der Synthese von Agrochemikalien und Pharmazeutika deshalb verbietet. Des Weiteren wurde gefunden (vgl. Vergleichsbeispiel 3, Tabelle 2), dass Esterasen für den Einsatz in Solvolyse-Reaktionenungeeignet sind, da diese entweder mangelhafte Selektivitäten (Selektivitäten E ≤ 2) und/oder kaum messbare Umsätze (TOF = 0) liefern.

Die von Yano et al. in US 5604120 beschriebene asymmetrische Hydrolyse racemischer endo-2-Acyloxynorbornane (wobei der Acyloxysubstituent durch geradkettige, verzweigte, cyclische, aliphatische oder aromatische Kohlenwasserstoffe gebildet wird) wiederum, liefert unter Verwendung der Lipase aus *candida antarctica* bestenfalls Enantioselektivitäten E von kleiner 50 und TOF kleiner 0.3 mmol g⁻¹h⁻¹. Bei der vergleichbaren lipasenkatalysierten Hydrolyse des Benzoylesters von 1,2-Isopropylidenglycerol nach Bianchi et al. in EP 556909 A1 werden Selektivitäten E zwischen 1.6 und maximal 7.9 erreicht.

Ein weiterer Vorteil der Verwendung von Diketen als Acylierungsmittel gegenüber der Umesterung von Vinylestern ist, dass Eutomer und Distomer durch einfache physikalische Methoden getrennt werden können. Die bei der Reaktion entstehenden Acetessigester unterscheiden sich in ihren physikalischen Eigenschaften (z. B. Siedepunkt, Löslichkeit der Ca²⁺-Salze) deutlich von den korrespondierenden Alkoholen und lassen sich so äußerst einfach (z. B. durch Destillation, Filtration) voneinander trennen, wohingegen der Vinylester und der ihm zugrund liegende Alkohol sich destillativ nur mit großem Aufwand trennen lassen. Der Verwendung von Acetessigestern ist deshalb gerade für die großtechnische Umsetzung einer Racematspaltung der Vorzug einzuräumen.

Eine optimale Racematspaltung sollte also vorteilhafterweise folgende Bedingungen erfüllen:
1. hohe Enantiomerenreinheit beider optischen Antipoden
2. hohe Selektivität des Enzyms
   (die Punkte 1 & 2 widerspiegeln eine hohe Enantioselektivität E)
3. hohe chemische Ausbeute
4. gute Raum-Zeit-Ausbeuten
5. einfache Trennung von Eutomer und Distomer
6. wirtschaftliche, großtechnische Umsetzbarkeit

Aufgabe der vorliegenden Erfindung ist es, ein weiteres enantioselektives Verfahren zur Herstellung sekundärer Alkohole zur Verfügung zu stellen, welches die aus dem Stand der Technik bekannten Nachteile vermeidet.

Die Aufgabe wurde gelöst durch ein Verfahren, in dem zu einem racemischen oder enatiomerenangereicherten Gemisch enthaltend enantiomere Acetessigester chiraler sekundärer Alkohole ein Nucleophil und eine Lipase gegeben wird und lediglich ein Enantiomer des Enantiomerengemisches solvolysiert wird. Auf diese Weise erhält man eine Mischung eines enantiomerenreinen oder enantiomerenangereicherten Alkohols und des korrespondierenden Acetessigesters, die leicht voneinander getrennt werden können.

Gegenstand der Erfindung ist ein enantioselektives Verfahren zur Herstellung von sekundären Alkoholen durch enantioselektive enzymatische Solvolyse eines racemischen oder enantiomerenangereicherten Gemisches der Acetessigsäureester der chiralen sekundären Alkohole in Gegenwart eines Nucleophils, dadurch gekennzeichnet, dass eine zur solvolytischen Spaltung einer Estergruppe befähigten Lipase verwendet wird.

Es wurde dabei überraschend gefunden, dass durch enzymatische Solvolyse deutlich höhere Enantioselektivitäten als durch die aus dem Stand der Technik bekannte umgekehrte Reaktion, die enzymatische Acylierung, erreicht werden können. Ferner tritt keine Enzymschädigung durch unerwünschte, reaktive Nebenprodukte auf, und Eutomer und Distomer können durch die einfache Abtrennung des entsprechenden enantiomerenreinen oder enantiomerenangereicherten Alkohols in Gegenwart des korrespondierenden Acetessigesters abgetrennt werden. Das erfindungsgemäße Verfahren erfüllt somit gleichermaßen die Erfordernisse an ein großtechnisch einfach zu realisierendes und wirtschaftlich attraktives Verfahren.

Die verwendeten racemischen oder enantiomerenangereicherten Substrate (Acetessigester) lassen sich, nach den aus dem Stand der Technik bekannten Verfahren, sehr einfach aus den korrespondierenden Alkoholen durch Umsetzung mit Diketen (Dimerisierungsprodukt von Keten) herstellen (s. a. Diketenes, Raimund Miller et al. in Ullmann's Encyclopedia of Industrial Chemistry, Vol A15, 5. Ed. (1990), S. 67-72). Die Acetessigester können dabei in hervorragender Reinheit und Ausbeute erhalten werden.

Das Prinzip des erfindungsgemäßen Verfahrens ist in der allgemeinen Gleichung (I) schematisch dargestellt, wobei die Reste R¹ und R², um ein chirogenes Kohlenstoffatom bei den zu Grunde liegenden Alkoholen zu erzeugen, verschieden sind und NuH allgemein für ein Nucleophil steht:

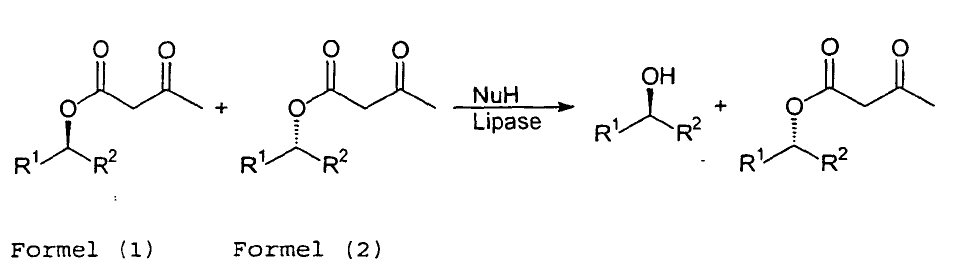

### Gleichung (I)

In einer bevorzugten Ausführungsform der Erfindung wird ein racemisches oder enantiomerenangereichertes Gemisch enthaltend Acetessigester der allgemeinen Formel (1) und (2) eingesetzt, wobei R¹ verschieden von R² und
die Reste R¹ und R² unabhängig voneinander ausgewählt werden aus der Gruppe enthaltend substituierte oder unsubstituierte C₆-C₁₈-Aryle, C₃-C₁₈-Heteroaryle, C₁-C₁₈-Alkyle, C₂-C₁₈-Alkenyle, C₂-C₁₈-Alkinyle, C₆-C₁₈-Aryl-C₁-C₁₈-Alkyle, C₃-C₁₈-Heteroaryl-C₁-C₁₈-Alkyle, C₆-C₁₈-Aryl-C₁-C₁₈-Alkenyle, C₃-C₁₈-Heteroaryl-C₁-C₁₈-Alkenyle, C₁-C₁₈-Alkoxy-C₁-C₁₈-Alkyle, C₁-C₁₈-Alkoxycarbonyle, Hydroxycarbonyl, C₁-C₁₈-Alkoxy-C₂-C₁₈-Alkenyle, C₆-C₁₈-Aryloxy-C₁-C₁₈-Alkyle, C₆-C₁₈-Aryloxy-C₂-C₁₈-Alkenyle, C₃-C₈-Cycloalkyle, C₃-C₈-Cycloalkyl-C₁-C₁₈-Alkyle, C₃-C₈-Cycloalkyl-C₂-C₁₈-Alkenyle
oder **R**^{**1**} und **R**^{**2**} zusammen mit dem Kohlenstoffatom, an das sie gebunden sind, ein asymmetrisches, substituiertes, unsubstituiertes oder Heteroatom-enthaltendes Cycloalkyliden bilden und
soweit es sich bei den Resten **R**^{**1**} und **R**^{**2**} um substituierte Reste handelt, werden diese Substituenten ausgewählt aus der Gruppe der gegebenenfalls wiederum substituierten Alkyl-, Alkenyl-, Alkinyl-, Aryl-, Heteroaryl-, Hydroxy-, Alkoxy-, Acyloxy-, Silyloxy-, Carboxylat-, Alkoxycarbonyl-, Amino-, Nitro- oder Halogenreste.

Soweit die vorstehend genannten Reste **R**^{**1**} und **R**^{**2**} ein Heteroatom enthalten, handelt es sich dabei vorzugsweise um O, N, S oder Si.
Bevorzugt handelt es sich bei den Resten **R**^{**1**} und **R**^{**2**} um Reste, die einen racemischen Alkohol mit einem Molgewicht < 300 Dalton bilden, da diese nach der Racematspaltung einfach zu trennende Eutomer-Distomer-Gemische ergeben und beispielsweise der sog. destillative Stress bei der Abtrennung des Alkohols vom verbleibenden Acetessigester vermieden werden kann.
Besonders bevorzugt sind racemische Alkohole, bei denen sich **R**^{**1**} und **R**^{**2**} deutlich in ihrem sterischen oder elektrochemischen Anspruch unterscheiden, da hier besonders hohe Selektivitäten erreicht werden können.

Besonders bevorzugte Resten **R**^{**1**} und **R**^{**2**} werden jeweils ausgewählt aus den folgende Reste enthaltenden Gruppen: In der Gruppe der C₆-C₁₈-Aryle aus: Phenyl, Naphthyl, Methylphenyl, Ethylphenyl, Propylphenyl, 1-Methylethylphenyl, Butylphenyl, 1-Methylpropylphenyl, 2-Methylpropylphenyl, 1,1-Dimethylethylphenyl, Methylnaphthyl, Ethylnaphthyl, Propylnaphthyl, 1-Methylethylnaphthyl, Butylnaphthyl, 1-Methylpropylnaphthyl, 2-Methylpropylnaphthyl, 1,1-Dimethylethylnaphthyl, Anthracyl, Phenanthryl, o-Nitrophenyl, m-Nitrophenyl, o-Chlorphenyl, m-Chlorphenyl, o-Bromphenyl, m-Bromphenyl, 2,4-Dichlorphenyl, 2,3-Dichlorphenyl, 3,5-Dichlorphenyl, 3,4-Dichlorphenyl, 2,4-Dimethoxyphenyl, 2,3-Dimethoxyphenyl, 3,5-Dimethoxyphenyl, 3,4-Dimethoxyphenyl, 2-Chlor-4-Nitrophenyl, o-Methoxyphenyl, m-Methoxyphenyl, p-Chlorphenyl, p-Methylphenyl, p-Ethylphenyl, p-Propylphenyl, p-Isopropylphenyl, p-tert-Butylphenyl, o-Methylphenyl, o-Ethylphenyl, o-Propylphenyl, o-Isopropylphenyl, o-tert-Butylphenyl, 4-Chlor-2-Methylphenyl, p-Vinylphenyl, 4-Methoxy-2-Methylphenyl, o-Methoxyphenyl, m-Methoxyphenyl oder p-Methoxyphenyl

In der Gruppe der C₃-C₁₈-Heteroaryle aus: 2-Indolyl, 3-Indolyl, 2-(4-Chlor)thienyl, 2-Pyrrolyl, 3-Pyrrolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 2-Pyridyl, 3-Pyridyl, 4-Pyridyl, 2-Furyl, 3-Furyl, 2-Thienyl, 3-Thienyl, 2-Thiazolyl, 4-Thiazolyl, 5-Thiazolyl, 6-Pyridazinyl,
C₁-C₁₈-Alkyle: Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Dekyl, Chlormethyl, Dichlormethyl, Trichlormethyl, Brommethyl, Nitromethyl, Hydroxymethyl, Chlorethyl, Bromethyl, Iodethyl, Aminoethyl, Monomethylaminoethyl, Dimethylaminoethyl, Trimethylsilyloxymethyl, Triethylsilyloxymethyl, Isopropyldimethylsilyloxymethyl, Norbornyldimethylsilyloxymethyl, Dinorbornylmethylsilyloxymethyl, Triisopropylsilyloxymethyl, tert.-Butyldimethylsilyloxymethyl, tert.-Butyldiphenylsilyloxymethyl, 2-Chlorethyl, 2-Nitroethyl, 2-Hydroxyethyl, 2-Trimethylsilyloxyethyl, 2-Isopropyldimethylsilyloxyethyl, 2-Norbornyldimethylsilyloxyethyl, 2-Dinorbornylmethylsilyloxyethyl, 2-Triisopropylsilyloxyethyl, 2-tert.-Butyldimethylsilyloxyethyl, 2-tert.-Butyldiphenylsilyloxyethyl, Hydroxycarbonylmethyl, Methoxycarbonylmethyl, Ethoxycarbonylmethyl, Phenyloxycarbonylmethyl, 1-Hydroxycarbonyl-1-chlormethyl, 1-Methoxycarbonyl-1-methyl, 1-Ethoxycarbonyl-1-methyl, Acyloxymethyl, Acyloxyethyl, Ethylcarbonyloxymethyl oder Propylcarbonyloxymethyl.

In der Gruppe der C₂-C₁₈-Alkenyle aus: Ethenyl (Vinyl), Propenyl, 1-Butenyl, 2-Butenyl, 3-Butenyl, 2-Methylpropenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1-Heptenyl, 2-Heptenyl, 3-Heptenyl, 4-Heptenyl, 5-Heptenyl, 6-Heptenyl, 1-Octenyl, 2-Octenyl, 3-Octenyl, 4-Octenyl, 5-Octenyl, 6-Octenyl, 7-Octenyl, 1-Nonenyl, 2-Nonenyl, 3-Nonenyl, 4-Nonenyl, 5-Nonenyl, 6-Nonenyl, 7-Nonenyl, 8-Nonenyl, 1-Decenyl, 2-Decenyl, 3-Decenyl, 4-Decenyl, 5-Decenyl, 6-Decenyl, 7-Decenyl, 8-Decenyl, 9-Decenyl, 2-Chlorvinyl, 3-Chlor-1-propenyl, 4-Chlor-1-Butenyl, 5-Chlor-1-Pentenyl, 6-Chlor-1-Hexenyl, 7-Chlor-1-Heptenyl, 8-Chlor-1-Octenyl, 9-Chlor-1-Nonenyl, 10-Chlor-1-Decenyl, 2-Nitrovinyl, 3-Nitro-1-propenyl, 4-Nitro-1-Butenyl, 5-Nitro-1-Pentenyl, 6-Nitro-1-Hexenyl, 7-Nitro-1-Heptenyl, 8-Nitro-1-Octenyl, 9-Nitro-1-Nonenyl, 2-Propenyl, 2-Butenyl, 1-Methyl-2-propenyl, 2-Methyl-2-propenyl, 10-Nitro-1-Decenyl, 1,2-Dimethyl-, 2-Hexenyl, 1-Ethyl-2-butenyl

In der Gruppe der C₂-C₁₈-Alkinyle aus: Ethinyl, Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl, 4-Methyl-pent-2-in-5-yl, 3-Chlor-1-propinyl, 4-Chlor-1-butinyl, 5-Chlor-1-pentinyl, 6-Chlor-1-hexinyl, 7-Chlor-1-heptinyl, 8-Chlor-1-octinyl, 9-Chlor-1-noninyl, 10-Chlor-1-decinyl, 2-Nitrovinyl, 3-Nitro-1-propinyl, 4-Nitro-1-butinyl, 5-Nitro-1-pentinyl, 6-Nitro-1-hexinyl, 7-Nitro-1-heptinyl, 8-Nitro-1-octinyl, 9-Nitro-1-noninyl, 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1, 1-Dimethyl-2-propinyl, 1-Ethyl-2-propinyl, 2-Hexinyl, 3-Hexinyl, 4-Hexinyl, 5-Hexinyl, 10-Nitro-1-decinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl oder 1-Ethyl-1-methyl-2-propinyl.

In der Gruppe der C₆-C₁₈-Aryl-C₁-C₁₈-Alkyle aus: Benzyl, Naphthylmethyl, o-Nitrophenylmethyl, m-Nitrophenylmethyl, o-Chlorphenylmethyl, m-Chlorphenylmethyl, 2,4-Dichlorphenylmethyl, 2,3-Dichlorphenylmethyl, 3,5-Dichlorphenylmethyl, 3,4-Dichlorphenylmethyl, 2,4-Dimethoxyphenylmethyl, 2,3-Dimethoxyphenylmethyl, 3,5-Dimethoxyphenylmethyl, 3,4-Dimethoxyphenylmethyl, 2-Chlor-4-Nitrophenylmethyl, o-Methoxyphenylmethyl, m-Methoxyphenylmethyl, p-Chlorphenylmethyl, p-Methylphenylmethyl, p-Ethylphenylmethyl, p-Propylphenylmethyl, p-Isopropylphenylmethyl, 4-Chlor-2-Methylphenylmethyl, p-Vinylphenylmethyl, Phenylethyl, Naphthylethyl, o-Nitrophenylethyl, m-Nitrophenylethyl, o-Chlorphenylethyl, m-Chlorphenylethyl, 2,4-Dichlorphenylethyl, 2,3-Dichlorphenylethyl, 3,5-Dichlorphenylethyl, 3,4-Dichlorphenylethyl, o-Methoxyphenylethyl, m-Methoxyphenylethyl, p-Chlorphenylethyl, p-Methylphenylethyl, p-Ethylphenylethyl, p-Propylphenylethyl oder p-Vinylphenylethyl.

In der Gruppe der C₃-C₁₈-Heteroaryl-C₁-C₁₈-Alkyle aus: 2-Indolylmethyl, 3-Indolylmethyl, 2-Pyrrolmethyl, 3-Pyrrolmethyl, 2-Pyridylmethyl, 3-Pyridylmethyl, 2-Furylmethyl, 3-Furylmethyl, 2-Imidazolylmethyl, 4-Imidazolylmethyl, 5-Imidazolylmethyl, 3-Pyridazinylmethyl, 4-Pyridazinylmethyl oder 5-Pyridazinylmethyl.

In der Gruppe der C₆-C₁₈-Aryl-C₁-C₁₈-Alkenyle aus: Styryl, 2-Phenylpropenyl, 1-Phenyl-1-butenyl, 2-Phenyl-1-butenyl, 3-Phenyl-1-butenyl, 1-Phenyl-2-butenyl, 2-Phenyl-2-butenyl, 3-Phenyl-2-butenyl, 1-Phenyl-3-butenyl, 2-Phenyl-3-butenyl, 3-Phenyl-3-butenyl, 1-Phenyl-1-pentenyl, 2-Phenyl-1-pentenyl, 3-Phenyl-1-pentenyl, 4-Phenyl-1-pentenyl, 1-Phenyl-2-pentenyl, 2-Phenyl-2-pentenyl, 3-Phenyl-2-pentenyl, 4-Phenyl-2-pentenyl, 1-Phenyl-3-pentenyl, 2-Phenyl-3-pentenyl, 3-Phenyl-3-pentenyl, 4-Phenyl-3-pentenyl, 1-Phenyl-4-pentenyl, 2-Phenyl-4-pentenyl, 3-Phenyl-4-pentenyl, 4-Phenyl-4-pentenyl, 1-Nitrophenyl-1-butenyl, 2-Nitrophenyl-1-butenyl, 3-Nitrophenyl-1-butenyl, 1-Nitrophenyl-2-butenyl, 2-Nitrophenyl-2-butenyl, 3-Nitrophenyl-2-butenyl, 1-Nitrophenyl-3-butenyl, 2-Nitrophenyl-3-butenyl, 3-Nitrophenyl-3-butenyl, 1-Methoxyphenyl-1-butenyl, 2-Methoxyphenyl-1-butenyl, 3-Methoxyphenyl-1-butenyl, 1-Methoxyphenyl-2-butenyl, 2-Methoxyphenyl-2-butenyl, 3-Methoxyphenyl-2-butenyl, 1-Methoxyphenyl-3-butenyl, 2-Methoxyphenyl-3-butenyl, 3-Methoxyphenyl-3-butenyl, 1-Chlorphenyl-1-butenyl, 2-Chlorphenyl-1-butenyl, 3-Chlorphenyl-1-butenyl, 1-Chlorphenyl-2-butenyl, 2-Chlorphenyl-2-butenyl, 3-Chlorphenyl-2-butenyl, 1-Chlorphenyl-3-butenyl, 2-Chlorphenyl-3-butenyl oder 3-Chlorphenyl-3-butenyl.

In der Gruppe der C₃-C₁₈-Heteroaryl-C₁-C₁₈-Alkenyle aus: 2-Indolylpropenyl, 1-Indolyl-1-butenyl, 2-Indolyl-1-butenyl, 3-Indolyl-1-butenyl, 1-Indolyl-2-butenyl, 2-Indolyl-2-butenyl, 3-Indolyl-2-butenyl, 1-Indolyl-3-butenyl, 2-Indolyl-3-butenyl, 3-Indolyl-3-butenyl, 2-Pyrrolylpropenyl, 1-Pyrrolyl-1-butenyl, 2-Pyrrolyl-1-butenyl, 3-Pyrrolyl-1-butenyl, 1-Pyrrolyl-2-butenyl, 2-Pyrrolyl-2-butenyl, 3-Pyrrolyl-2-butenyl, 1-Pyrrolyl-3-butenyl, 2-Pyrrolyl-3-butenyl, 3-Pyrrolyl-3-butenyl, 2-Pyrimidylpropenyl, 1-Pyrimidyl-1-butenyl, 2-Pyrimidyl-1-butenyl, 3-Pyrimidyl-1-butenyl, 1-Pyrimidyl-2-butenyl, 2-Pyrimidyl-2-butenyl, 3-Pyrimidyl-2-butenyl, 1-Pyrimidyl-3-butenyl, 2-Pyrimidyl-3-butenyl, 3-Pyrimidyl-3-butenyl, 2-Imidazolylpropenyl, 1-Imidazolyl-1-butenyl, 2-Imidazolyl-1-butenyl, 3-Imidazolyl-1-butenyl, 1-Imidazolyl-2-butenyl, 2-Imidazolyl-2-butenyl, 3-Imidazolyl-2-butenyl, 1-Imidazolyl-3-butenyl, 2-Imidazolyl-3-butenyl, 3-Imidazolyl-3-butenyl, 2-Pyridylpropenyl, 1-Pyridyl-1-butenyl, 2-Pyridyl-1-butenyl, 3-Pyridyl-1-butenyl, 1-Pyridyl-2-butenyl, 2-Pyridyl-2-butenyl, 3-Pyridyl-2-butenyl, 1-Pyridyl-3-butenyl, 2-Pyridyl-3-butenyl oder 3-Pyridyl-3-butenyl.

In der Gruppe der C₁-C₁₈-Alkoxy-C₁-C₁₈-Alkyle aus: Methoxymethyl, Ethoxymethyl, Methoxyethyl, Ethoxyethyl oder Benzyloxymethyl.

In der Gruppe der C₁-C₁₈-Alkoxy-C₂-C₁₈-Alkenyle aus: Methoxybutenyl, Ethoxybutenyl oder Benzyloxybutenyl.
In der Gruppe der C₆-C₁₈-Aryloxy-C₁-C₁₈-Alkyle : Phenyloxymethyl.

In der Gruppe der C₆-C₁₈-Aryloxy-C₂-C₁₈-Alkenyle: Phenyloxybutenyl.

In der Gruppe der C₃-C₈-Cycloalkyle aus: Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclypropyl, 1-Methyl-2-Ethylcyclopropyl, Cyclooctyl, Cyclononyl, Cyclodecyl, Cyclopropenyl, Cyclobutenyl, Cyclopentenyl, Cyclohexenyl, Cycloheptenyl oder Cyclooctenyl.

In der Gruppe der C₃-C₈-Cycloalkyl-C₁-C₁₈-Alkyle aus: Cyclopropylmethyl, Cyclobutylmethyl, Cyclopentylmethyl, Cyclohexylmethyl.

In der Gruppe der C₃-C₈-Cycloalkyl-C₂-C₁₈-Alkenyle: Cyclopropyl-1-butenyl, Cyclobutyl-1-butenyl, Cyclopentyl-1-butenyl, Cyclobutenyl-1-butenyl oder Cyclopentenyl-1-butenyl.

In der Gruppe der C₁-C₁₈-Alkoxycarbonyl: Methoxycarbonyl, Ethoxycarbonyl oder Benzyloxycarbonyl.

In der Gruppe der Cycloalkylidene aus: 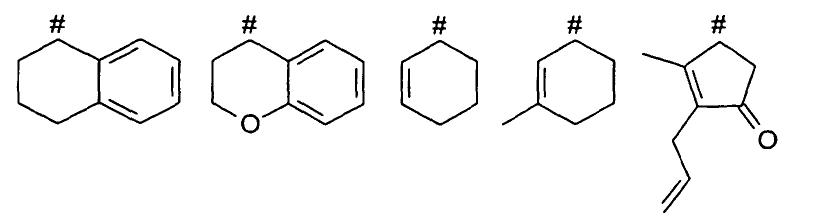 wobei # jeweils den Anknüpfpunkt des Alkylidens markiert.

Als besonders bevorzugte Substrate seine die Acetessigester folgender racemischer Alkohole genannt:
2-Butanol, 2-Pentanol, 2-Hexanol, 2-Heptanol, 2-Oktanol, 1-Phenylethanol, 1-Phenyl-1-propanol, 1-Phenyl-2-propanol, 1-(4-Chlorphenyl)ethanol, 1-(4-Chlorphenyl)propanol, 2-Chlor-1-phenylethanol, 3-Chlor-1-Phenylpropanol, 2-Chlor-1-(4-Chlorphenyl) ethanol, 3-Chlor-1-(4-Chlorphenyl)propanol, 2-Chlor-1- (3-Chlorphenyl) ethanol, 3-Chlor-1- (3-Chlorphenyl)propanol, 2-Chlor-1-(2-Chlorphenyl)ethanol, 3-Chlor-1- (2-Chlorphenyl)propanol, 1- (4-Nitrophenyl) ethanol, 1- (4-Nitrophenyl)propanol, 1-Naphthylethanol, 1-Naphthylpropanol, 1-(6-Methoxynaphthyl)ethanol, 1-(6-Methoxynaphthyl)propanol, 2-Chlor-1-naphthylethanol, 3-Chlor-1-naphthylpropanol, 2-Chlor-1-(6-methoxynaphthyl)ethanol, 3-Chlor-1-(6-methoxynaphthyl)propanol, 1- (4-Methylphenyl) ethanol, 1-(4-Methylphenyl)propanol, 2-Chlor-1-(4-Methylphenyl)ethanol, 3-Chlor-1-(4-Methylphenyl)propanol, 1-(4-Ethylphenyl)ethanol, 1-(4-Ethylphenyl)propanol, 2-Chlor-1-(4-ethylphenyl)ethanol, 3-Chlor-1-(4-ethylphenyl)propanol, 1-(4-Methoxyphenyl)ethanol, 1-(4-Methoxyphenyl)propanol, 2-Chlor-1-(4-methoxyphenyl)ethanol, 3-Chlor-1-(4-methoxyphenyl)propanol, 1-(2-Methylphenyl)ethanol, 1-(2-Methylphenyl)propanol, 2-Chlor-1- (2-Methylphenyl) ethanol, 3-Chlor-1-(2-Methylphenyl)propanol, 1-(2-Ethylphenyl)ethanol, 1-(2-Ethylphenyl)propanol, 2-Chlor-1-(2-ethylphenyl)ethanol, 3-Chlor-1-(2-ethylphenyl)propanol, 1-(2-Methoxyphenyl)ethanol, 1-(2-Methoxyphenyl)propanol, 2-Chlor-1-(2-methoxyphenyl)ethanol, 3-Chlor-1-(2-methoxyphenyl)propanol, 1-(3-Methylphenyl)ethanol, 1-(3-Methylphenyl)propanol, 2-Chlor-1-(3-Methylphenyl)ethanol, 3-Chlor-1-(3-Methylphenyl)propanol, 1-(3-Ethylphenyl)ethanol, 1-(3-Ethylphenyl)propanol, 2-Chlor-1-(3-ethylphenyl)ethanol, 3-Chlor-1-(3-ethylphenyl)propanol, 1-(3-Methoxyphenyl)ethanol, 1-(3-Methoxyphenyl)propanol, 2-Chlor-1-(3-methoxyphenyl)ethanol, 3-Chlor-1-(3 -methoxyphenyl) propanol, 1- (1,3) - Benzodioxolethanol, 5-Phenylpent-1-en-3-ol, 4-Phenylbut-3-en-2-ol, 1-(6-Methoxy-2-naphthyl)ethanol, 1-(4-Isobutylphenyl) ethanol, 1-Pyridin-3-ylethanol, 1-(2-Furyl) ethanol, 2-Bromo-1-(4-nitrophenyl)ethanol, Cyclopropyl(phenyl)methanol, 3-Chloro-1-thien-2-ylpropan-1-ol, 3-Iodo-1-thien-2-ylpropan-1-ol, 3-(Methylamino)-1-thien-2-ylpropan-1-ol, 3-(Dimethylamino)-1-thien-2-ylpropan-1-ol, Methyl 3-ethyl-3-(hydroxymethyl)heptanoate, 1-Trimethylsilanyloxy-propan-2-ol, 1-Triisopropylsilanyloxypropan-2-ol, 1-(tert-Butyl-dimethyl-silanyloxy)-propan-2-ol, 1-(tert-Butyl-diphenyl-silanyloxy)-propan-2-ol, 1-(tert-Butyldiphenyl-silanyloxy)-butan-2-ol, 1-(tert-Butyl-diphenylsilanyloxy)-3-chloro-propan-2-ol, 1-(2-Norbornyl-dimethylsilanyloxy)-propan-2-ol, 1-(2-Norbornyl-dimethyl-silanyloxy)-3-chloro-propan-2-ol, 1,2,3,4-Tetrahydro-naphthalen-1-ol, Chroman-4-ol, Cyclohex-2-enol, 3-Methyl-cyclohex-2-enol, 2-Allyl-4-hydroxy-3-methyl-cyclopent-2-enone, Methyl 3-hydroxybutanoat, Ethyl 3-hydroxybutanoat, Methyl 4-chloro-3-hydroxybutanoat oder Ethyl 4-chloro-3-hydroxybutanoat.

Die für das erfindungsgemäße Verfahren als Edukte einzusetzenden Acetessigester sekundärer Alkohole werden bevorzugt durch Umsetzung von racemischen oder enatiomerenangereicherten Mischungen der sekundären Alkohole mit Diketen nach den aus dem Stand der Technik bekannten Verfahren erhalten.

Allgemein können für das erfindungsgemäße Verfahren Nucleophile der allgemeinen Formel NuH eingesetzt werden, wobei Nu OR⁵, SR⁵, oder NR⁶R⁷ bedeuten kann und
**R**^{**5**} ausgewählt wird aus der Gruppe enthaltend Wasserstoff, substituiertes oder unsubstituiertes C₁-C₁₈-Alkyl, C₂-C₁₈-Alkenyl, C₂-C₁₈-Alkinyl, C₆-C₁₈-Aryl-C₁-C₁₈-Alkyl, C₃-C₁₈-Heteroaryl-C₁-C₁₈-Alkyl, C₆-C₁₈-Aryl-C₂-C₁₈-Alkenyl, C₃-C₁₈-Heteroaryl-C₂-C₁₈-Alkenyl und
**R**^{**6**} und **R**^{**7**} gleich oder verschieden sein können und unabhängig voneinander ausgewählt werden aus der Gruppe enthaltend Wasserstoff, substituiertes oder unsubstituiertes C₁-C₁₈-Alkyl, C₂-C₁₈-Alkenyl, C₂-C₁₈-Alkinyl-C₆-C₁₈-Aryl, C₃-C₁₈-Heteroaryl, C₆-C₁₈ -Aryl-C₁-C₁₈ -Alkyl, C₃-C₁₈-Heteroaryl-C₁-C₁₈-Alkyl, C₆-C₁₈-Aryl-C₂-C₁₈-Alkenyl, C₃-C₁₈-Heteroaryl-C₂-C₁₈-Alkenyl und
soweit es sich bei den Resten R⁵, R⁶ und R⁷ um substituierte Reste handelt, werden diese Substituenten ausgewählt aus der Gruppe der gegebenenfalls wiederum substituierten Alkyl-, Alkenyl-, Alkinyl-, Aryl-, Heteroaryl-, Hydroxy, Alkoxy-, Acyloxy, Silyloxy, Carboxylat-, Alkoxycarbonyl-, Amino-, Nitro- oder Halogenreste.

Soweit die vorstehend genannten Reste **R**^{**5**}**, R**^{**6**} und R⁷ ein Heteroatom enthalten, handelt es sich dabei vorzugsweise um 0, N, S oder Si.

In einer bevorzugten Ausführungsform des erfindungsgemäßen Verfahrens steht das Nucleophil NuH für eine sauerstoffhaltige Verbindung HOR⁵.

Besonders bevorzugt handelt es sich dabei um einen verzweigten oder unverzweigten C₁-C₆-Alkohol oder Wasser (**R**^{**5**} = H) , insbesondere Methanol, Ethanol, n-Propanol, iso-Propanol oder Butanol.

Für das erfindungsgemäße Verfahren sind prinzipiell alle Lipasen, die zur Spaltung einer Esterbindung befähigt sind, geeignet. Bevorzugt handelt es sich um eine Lipase der Klasse 3.1.1.3 gemäß Internationaler Enzym-Nomenklatur, Committee of the International Union of Biochemistry and Molecular Biology. Wegen ihrer einfacheren Zugänglichkeit besonders bevorzugt handelt es sich um Lipasen mikrobiellen Ursprungs. Als Lipasen mikrobiellen Ursprungs seien beispielsweise Lipasen aus Pilzen, Hefen oder Bakterien wie beispielsweise von Alcaligenes sp. (BTL2), Aspergillus niger (ANL), Aspergillus oryzae, Bacillus sp., Bacillus stearothermophilus, Bacillus thermoglucosidasius, Candida antarctica (CAL), Candida lipolytica (CLL), Candida rugosa (CRL), Chromobacterium viscosum (CVL), Geotrichum scandium (GCL), Mucor miehei (MML), Penicillium camembertii (PcamL), Penicillium roquefortii (ProqL), Pseudomonas cepacia (PCL), Pseudomonas fluorescens (PFL), Pseudomonas sp. (PSL), Rhizomucor javanicus (RJL), Rhizopus arrhizus (RAL), Rhizopus niveus (RNL), Saccharomyces cerevisiae, Thermoanaerobium brockii, Thermomyces lanuginosa (TLL) genannt.

Besonders bevorzugt werden Lipasen aus Candida-Arten wie zum Beispiel Candida antarctica B (CAL-B).

Als Enzym ganz besonders bevorzugt sind Novozym® 435, 525 (Firma Novo, Dänemark) und Chirazyme® L2 (Firma Böhringer Mannheim, Deutschland).

Die Enzyme werden in der Reaktion direkt oder in immobilisierter Form (als Immobilisate) an unterschiedlichste unlösliche Träger in adsorptiv oder kovalent gebundener Form eingesetzt. Die Immobilisate können hergestellt werden durch Lösen des Enzyms in einem Puffer bei geeignetem pH und anschließender passiver Adsorption an den Träger wie z. B. Diatomeenerde (Celite®), Aktivkohle, Aluminiumoxid, Kieselgel, Kieselguhr, monodispers lösliche Organosiloxanpartikel oder Harze (z. B. Amberlite®, Dowex®). Alternativ können die Enzyme auch kovalent an den Träger gebunden werden (z. B. Polystyrol oder Epoxy-Harze wie Eupergit®). Die geträgerten Enzyme können durch Lyophilisieren getrocknet werden.

Die im erfindungsgemäßen Verfahren einzusetzende Menge an Lipase hängt allgemein von der Art des Edukts, des Produkts und der Aktivität der Enzympräparation ab. Die für die Reaktion optimale Enzymmenge kann vom Fachmann leicht durch einfache Vorversuche ermittelt werden.

Je nach Lipase liegt das Enzym-Substratverhältnis berechnet als Molverhältnis zwischen Enzym und Substrat im Allgemeinen zwischen 1 : 1.000 bis 1 : 50.000.000, bevorzugt bei 1 : 10.000 bis 1 : 5.000.000.

Die Enantioselektivität E der Lipasen liegt dabei in der Regel zwischen 5 und > 100. Bevorzugt ist die Enantioselektivität größer als 10.

In dem erfindungsgemäßen Verfahren kann das Lösungsmittel direkt als Nucleophil (NuH) fungieren. Alternativ können auch Mischungen des Nucleophils (NuH) mit einem oder mehreren aprotischen oder protogenen Lösungsmitteln eingesetzt werden, sofern die Lösungsmittel oder Lösungsmittelgemische die Reaktivität des Enzyms nicht beeinflussen oder zu unerwünschten Nebenreaktionen führen.
Vorteilhafterweise wird die Reaktion in einer Mischung aus dem Nucleophil (NuH) und einem geeigneten inerten Lösungsmittel durchgeführt.

Das inerte Lösungsmittel wird bevorzugt ausgewählt aus der Gruppe enthaltend aliphatische Kohlenwasserstoffe, aromatische Kohlenwasserstoffe, halogenierte Kohlenwasserstoffe, Ether, Alkohole, die kein Nucleophil im Sinne der o. g. enzymatischen Reaktion darstellen, Ester oder Acetonitril oder Mischungen der genannten Lösungsmittel.

Aus der Gruppe der Kohlenwasserstoffe ist insbesondere Hexan, Cyclohexan, Petrolether oder Toluol bevorzugt.

Aus der Gruppe der halogenierten Kohlenwasserstoffe ist Methylenchlorid oder Chloroform bevorzugt.

Aus der Gruppe der Ether ist Methyl-tert.-Butylether (MTBE), THF, Diethylether, Diisopropylether oder Dioxan bevorzugt.

Alkohole, die kein Nucleophil im Sinne der o. g. enzymatischen Reaktion darstellen, sind insbesondere tertiäre Alkohole wie tert.-Butanol.

Ein besonders bevorzugtes Reaktionsmedium ist eine Mischung aus Wasser, n-Propanol oder Isopropanol (als Nucleophil NuH) und einem inerten Lösungsmittel, besonders der oben genannten bevorzugten Lösungsmittel, insbesondere MTBE.

Das Verhältnis Nucleophil zu Lösungsmittel (v/v) liegt dabei vorzugsweise in einem Bereich von 1 : 10.000 bis 10.000 zu 1.

Besonders bevorzugt sind Mischungen des Nucleophils mit aprotischen Lösungsmitteln, wie Methyl-tert.-Butylether (MTBE) oder Diisopropylether in einem Verhältnis Nucleophil/Lösungsmittel (v/v) von 1 : 100 bis 100 : 1.

Wird als Nucleophil Wasser verwendet (Nu = OH), so kann, um einen vorgegebenen pH-Wert einzuhalten, dieser durch Zugabe eines Puffers eingestellt und konstant gehalten werden. Bevorzugt wird dabei ein Na₂HPO₄ / NaH₂PO₄-Puffer mit einem pH-Wert von 7.0. Zum selben Zweck kann auch eine wässrige Lauge, bevorzugt die Lösung eines Alkalihydroxyds in Wasser, besonders bevorzugt die wässrige Lösung von NaOH oder KOH, zudosiert werden.

Die Reaktion wird vorteilhafterweise bei einer Temperatur zwischen 0 °C und 75 °C durchgeführt, bevorzugt zwischen 10 °C und 60 °C, besonders bevorzugt zwischen 20 °C und 50 °C.

Die Reaktionszeiten betragen je nach Substrat, Ester und Enzymart und -menge zwischen 10 Minuten bis 7 Tage. Bevorzugt liegen die Reaktionszeiten zwischen 1 bis 48 Stunden.

Der Reaktionsverlauf lässt sich leicht mit üblichen Methoden, wie beispielsweise über GC, HPLC oder den Laugenverbrauch bei der pH-Stat-Titration, verfolgen. Die Reaktion kann je nach gewünschtem Ergebnis (hohe Umsetzung, hoher Enantiomerenüberschuss des Substrats oder des Produkts) beendet werden. Im Idealfall ist die Reaktion bei einem Umsatz von 50 % bei einer hohen Enantiomerenreinheit sowohl im Substrat als auch im Produkt beendet.

Vorzugsweise wird die Reaktion beispielsweise durch Separation des Substrats oder Produkts vom Enzym, z. B. durch Extraktion, Filtration oder Destillation beendet. Der Abbruch der Reaktion kann auch durch Desaktivierung des Enzyms, z. B. durch thermische oder chemische Denaturierung erfolgen.

Falls die Reaktion durch wiederholtes, kontinuierliches Pumpen der Reaktionslösung durch einen mit Enzym gefüllten Behälter durchgeführt wird, wird die Reaktion vorzugsweise durch Beenden des Umpumpens beendet.

Je nach Enzym wird das (*R*)- bzw. (*S*)-Stereoisomer (siehe allgemeine Gleichung (I)) des Esters solvolysiert und der korrespondierende freie Alkohol selektiv gebildet. Das jeweils andere Enantiomer wird nicht umgesetzt und liegt weiterhin unverändert in Form seines Acetessigesters vor.

Das erhaltene Produktgemisch lässt wegen der signifikanten Siedepunktsunterschiede (vgl. Tabelle (1)) zwischen Ester und Alkohol destillativ einfach in Eutomer und Distomer auftrennen. Dies ermöglicht die quantitative Trennung der Produkte, was wiederum Voraussetzung für den Erhalt der optischen Reinheit ist. So kann in einer bevorzugten Ausführungsform der niedriger siedende Alkohol abdestilliert werden, wobei der Acetessigester im Sumpf verbleibt. Der Acetessigester kann anschließend in einer separaten Destillation oder nach erfolgter Hydrolyse gereinigt werden.

In Gleichung (II) (vgl. Beispiel 1) sei exemplarisch das Verfahren anhand der erfindungsgemäßen Umsetzung der racemischen Mischung des Acetessigesters von 1-Phenyl-ethanol zum (*R*)-Enantiomer von 1-Phenyl-ethanol (Ia) unter Erhalt des korrespondierenden Esters von (*S*)-1-Phenyl-ethanol (IIb) mit entgegengesetzter Chiralität und dessen weiterer Umsetzung in einem sich anschließenden Schritt bestehend aus Enantiomerentrennung und Verseifung zum (*S*)-1-Phenyl-ethanol (Ib) skizziert.

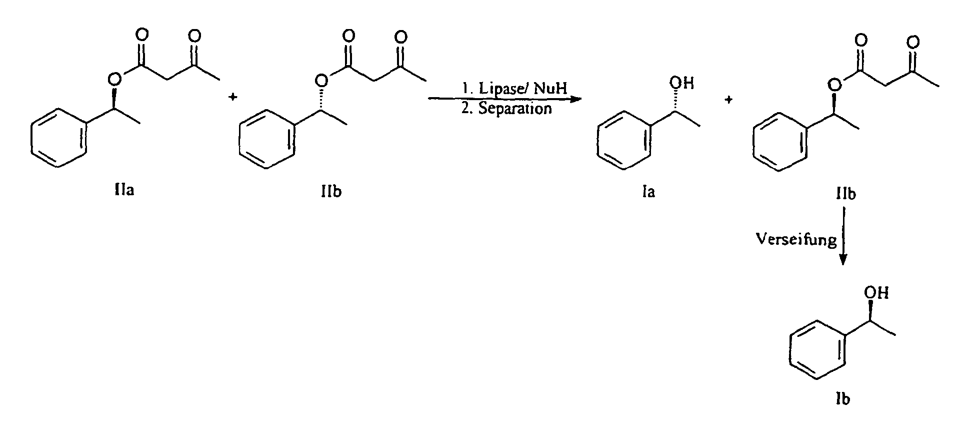

### Gleichung (II)

Die Fig. 1 zeigt den Reaktionsverlauf (Umsatz im Verlauf der Zeit) der CAL-B katalysierten Umsetzung von (*rac*)-1-Phenylethyl-3-Oxobutanoat zu (1*R*)-1-Phenylethanol und (1*S*)-1-Phenylethyl-3-Oxobutanoat.

Fig. 2 zeigt die Selektivität *E* der CAL-B katalysierten Umsetzung von (*rac*)-1-Phenylethyl-3-Oxobutanoat zu (1*R*)-1-Phenylethanol und (1*S*)-1-Phenylethyl-3-Oxobutanoat als Funktion des Enantiomerenüberschusses (ee) vom Umsatz (c)

### Beispiele

### Beispiel 1

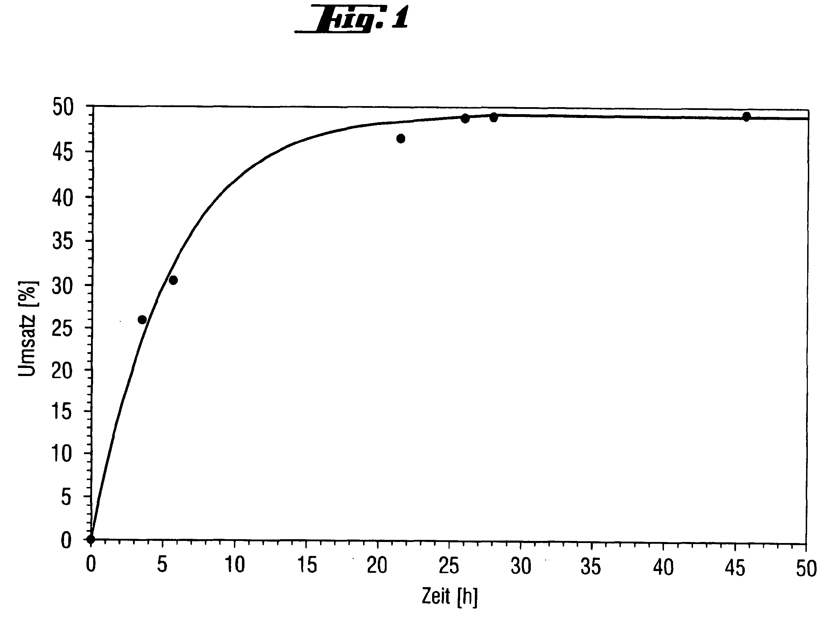

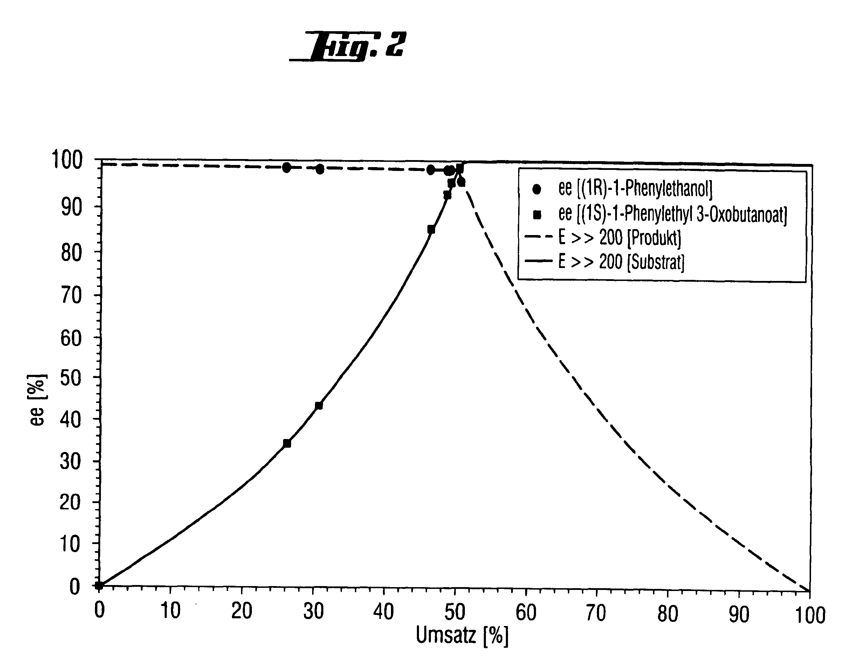

Eine Lösung von (*rac*)-1-Phenylethyl 3-Oxobutanoat (16 % (w/w)) in einer Mischung aus Isopropanol und MTBE (1:1 (v/v))) wird auf 40 °C thermostatisiert. Durch Zugabe von Novozym® 435 (3 % (w/w) bezogen auf das Racemat) wird die Reaktion gestartet. In regelmäßigen Abständen werden Proben entnommen und der ee von Substrat und Produkt gemessen bzw. der Umsatz bestimmt (siehe Fig. 1 und 2). Nachdem ein Umsatz von - 50 % erreicht ist, wird die Reaktion durch Abfiltrieren des Enzyms unterbrochen. Die organische Phase wird dann und unter vermindertem Druck eingeengt. Der Rückstand enthält (1*R*)-1-Phenylethanol (ee = 98%) und (1*S*)-1-Phenylethyl 3-Oxobutanoat (ee = 96%). Beide Verbindungen werden destillativ voneinander getrennt. (1*S*)-1-Phenylethyl 3-Oxobutanoat wird anschließend nach bekannten Verfahren verseift und man erhält (1*S*)-1-Phenylethanol als Produkt.

### Beispiel 2

Alle folgenden Beispiele (Tabelle (1) : 1a-h, 2a-e, 3a-1, 4a-1, 5a-f) dienen der weiteren Veranschaulichung der vorliegenden Erfindung und wurden entsprechend der folgenden allgemeinen Arbeitsvorschrift durchgeführt:
Eine Lösung des racemischen Acetessigesters (-16 % (w/w)) in einer Mischung aus dem entsprechenden Nucleophil (siehe Nu in Tabelle (1)) und gegebenenfalls einem Cosolvens (s. Tabelle (1)) wird auf 40 °C thermostatisiert. Durch Zugabe des entsprechenden Enzyms wird die Reaktion gestartet. Die Reaktion wird durch Abfiltrieren des Enzyms abgebrochen. Die organische Phase wird dann und unter vermindertem Druck eingeengt. Beide Verbindungen werden destillativ von einander getrennt.

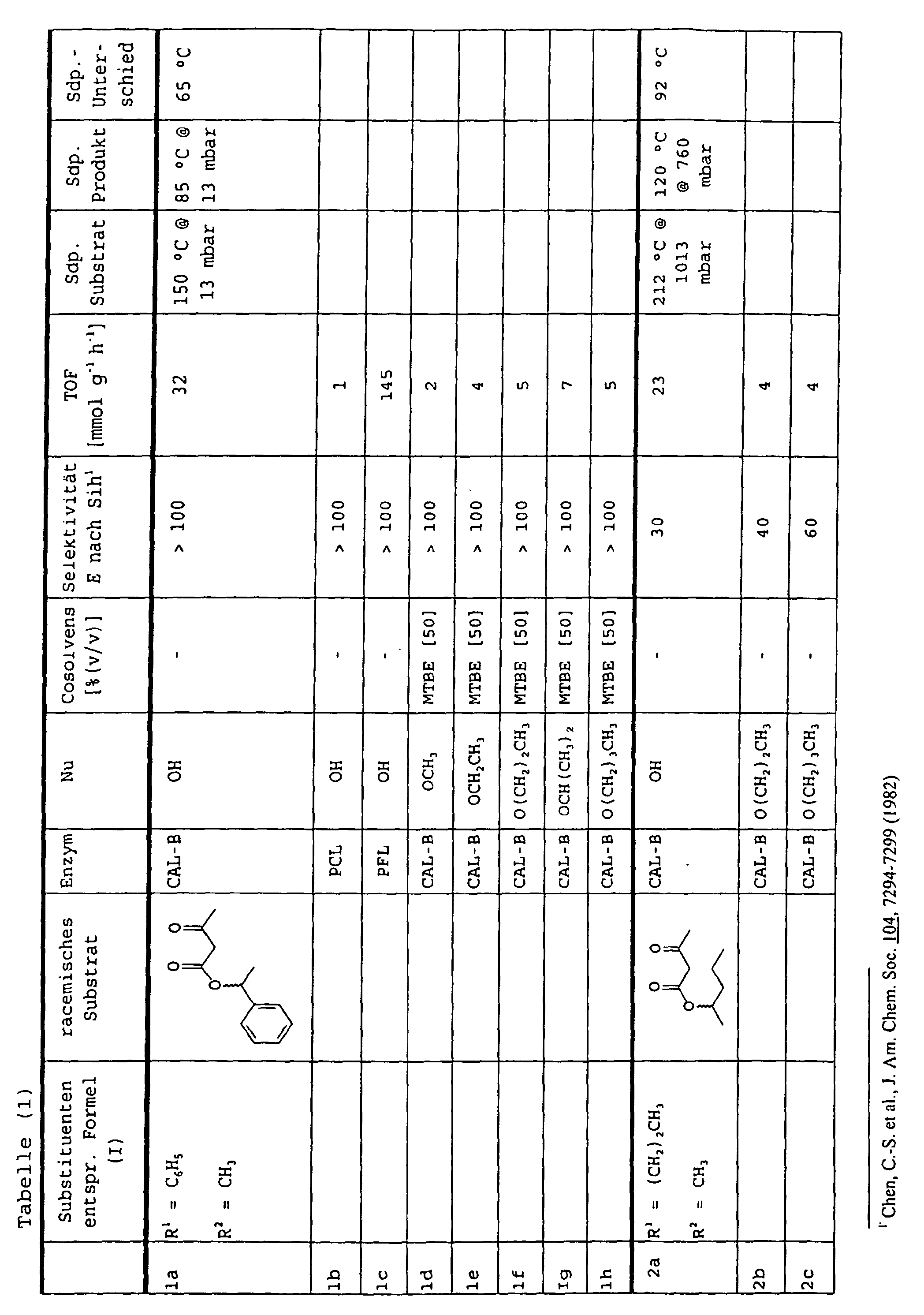

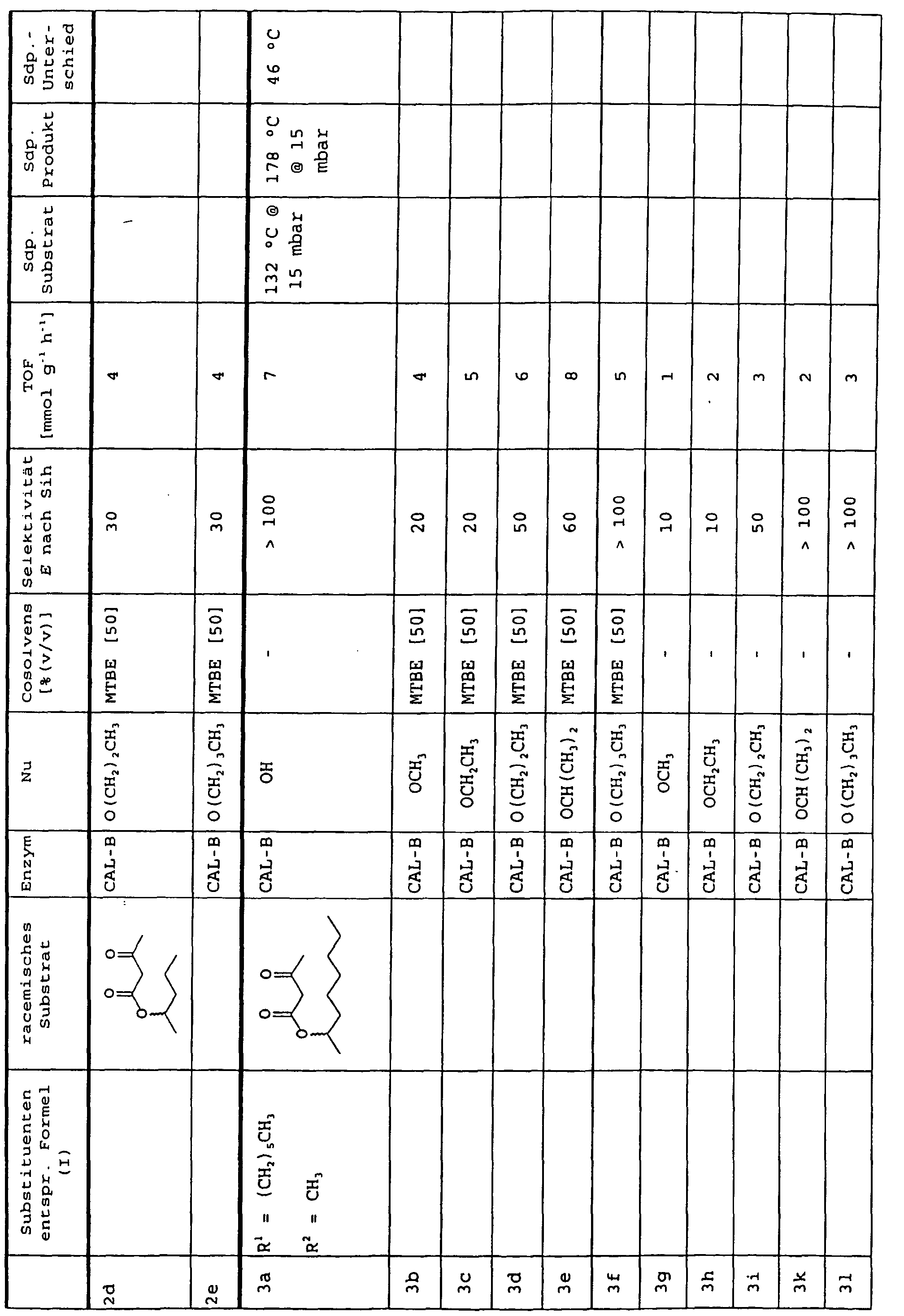

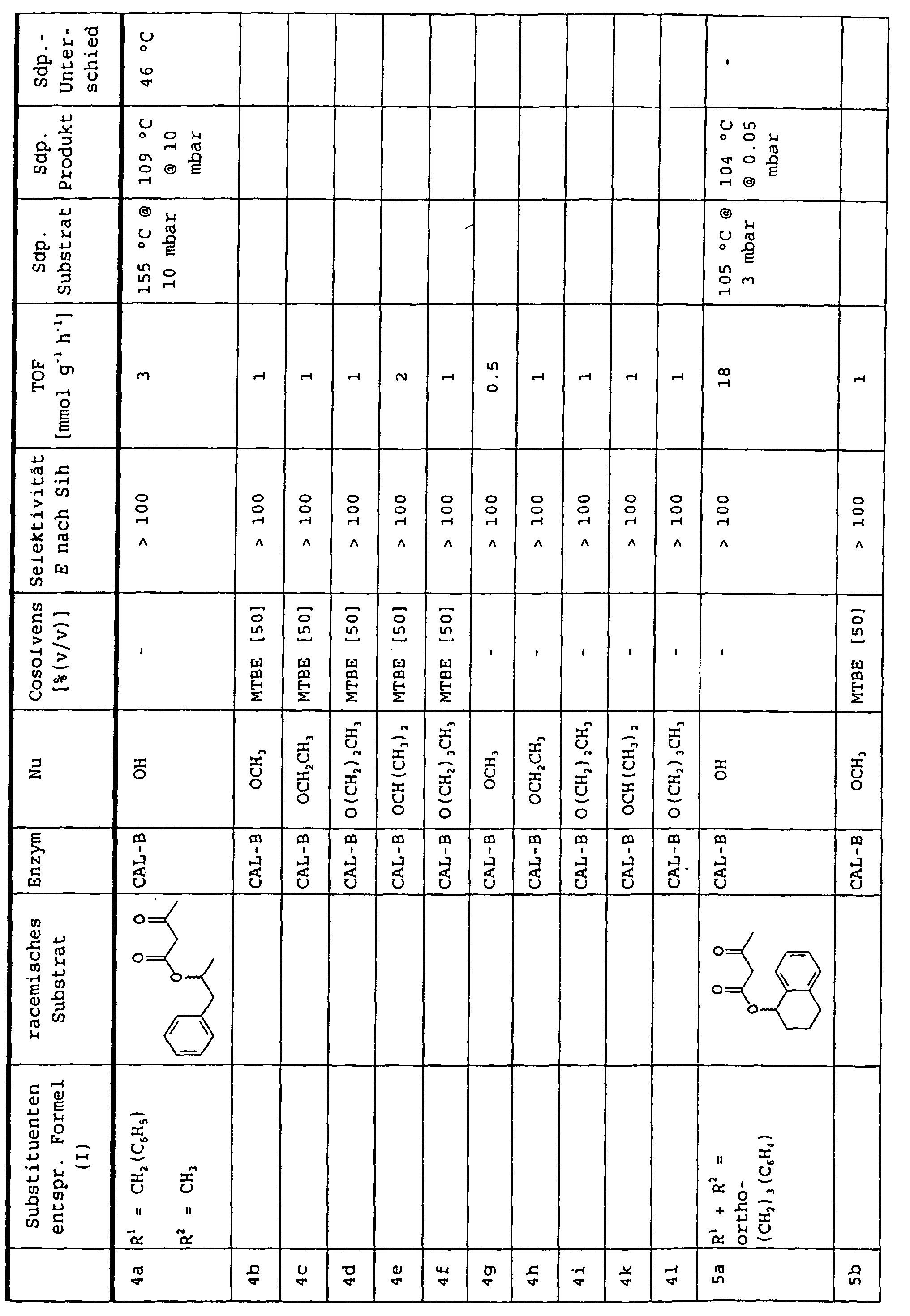

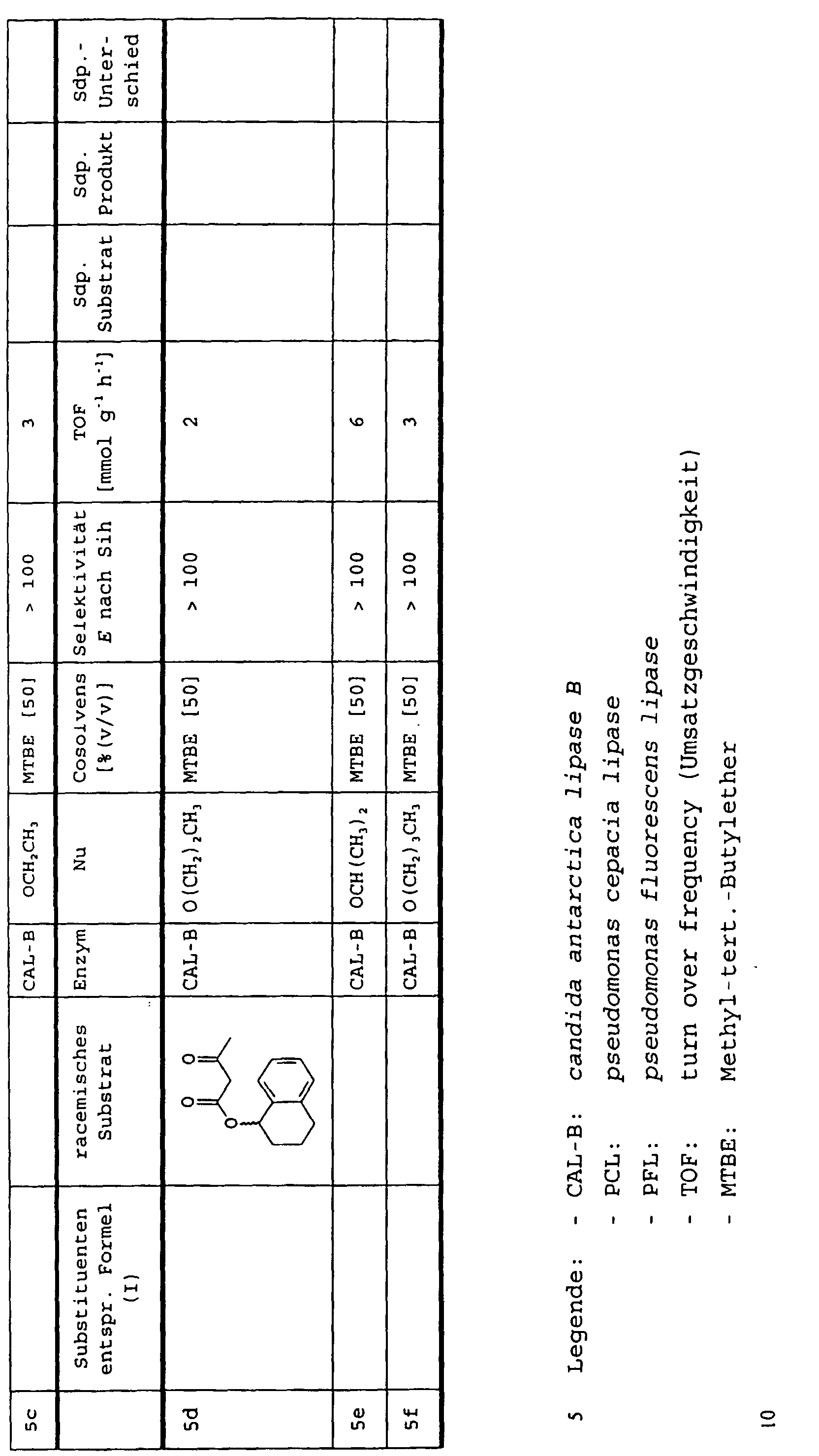

### Vergleichsbeispiel 3

Alle folgenden Beispiele (Tabelle (2): 6a-e) dienen der weiteren Veranschaulichung der vorliegenden Erfindung und wurden entsprechend der im Beispiel 2 aufgeführten allgemeinen Arbeitsvorschrift durchgeführt:

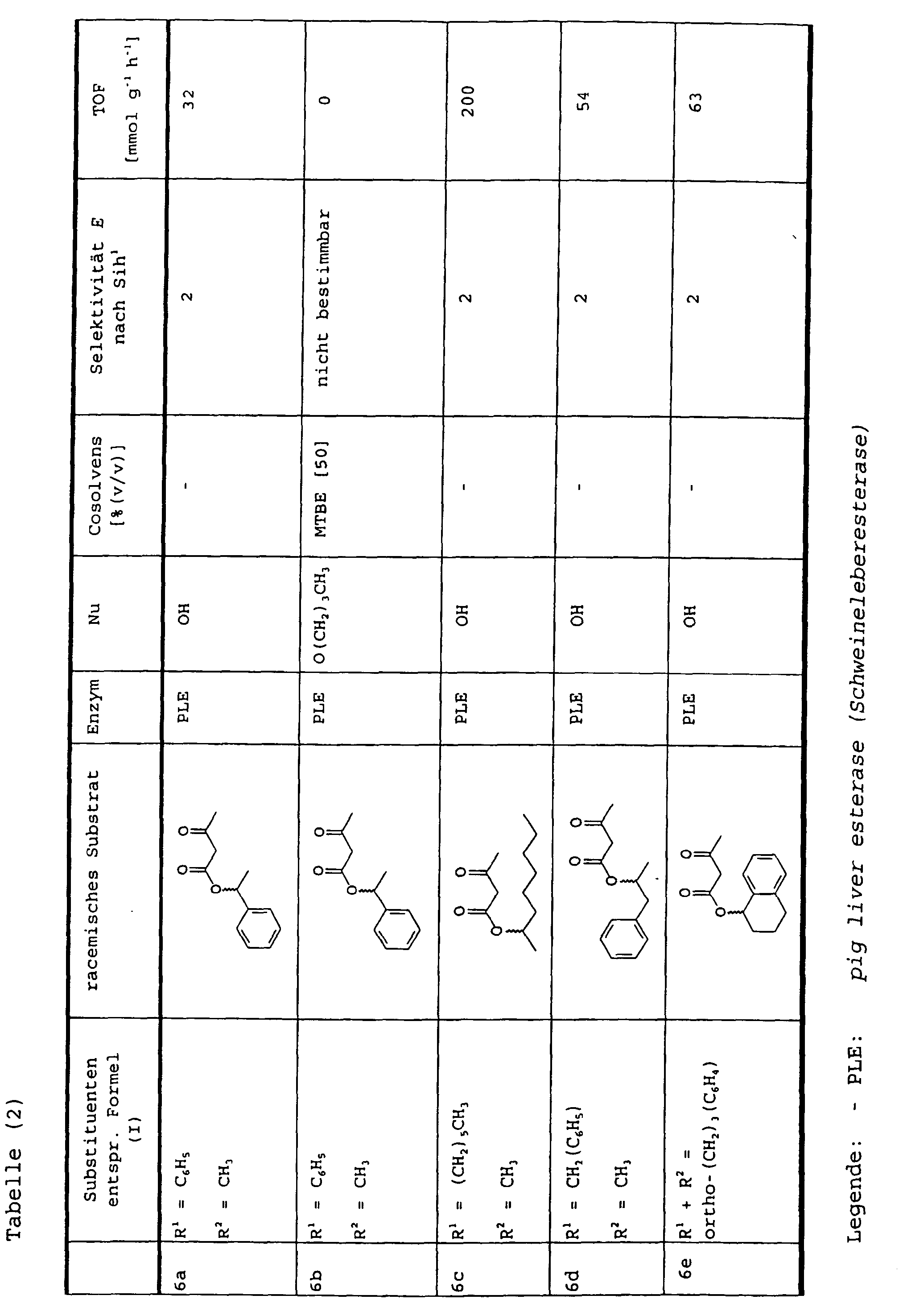

## Patentansprüche

1. Verfahren zur enantioselektiven Herstellung von sekundären Alkoholen durch enantioselektive enzymatische Solvolyse eines racemischen oder enantiomerenangereicherten Gemisches der Acetessigsäureester der chiralen sekundären Alkohole in Gegenwart eines Nucleophils, **dadurch gekennzeichnet, dass** eine zur solvolytischen Spaltung einer Estergruppe befähigte Lipase verwendet wird.

2. Verfahren nach Anspruch 1, **dadurch gekennzeichnet, dass** ein racemisches oder enantiomerenangereichertes Gemisch enthaltend Acetessigester der allgemeinen Formel (1) und (2) 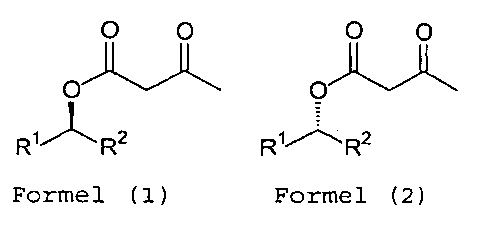 eingesetzt wird, wobei R¹ verschieden von R² ist und
die Reste R¹ und R² unabhängig voneinander ausgewählt werden aus der Gruppe enthaltend substituierte oder unsubstituierte C₆-C₁₈-Aryle, C₃-C₁₈-Heteroaryle, C₁-C₁₈-Alkyle, C₂-C₁₈-Alkenyle, C₂-C₁₈-Alkinyle, C₆-C₁₈-Aryl-C₁-C₁₈-Alkyle, C₃ -C₁₈ -Heteroaryl-C₁ -C₁₈-Alkyle, C₆-C₁₈ -Aryl-C₁-C₁₈-Alkenyle, C₃-C₁₈-Heteroaryl-C₁-C₁₈-Alkenyle, C₁-C₁₈-Alkoxy-C₁-C₁₈-Alkyle, C₁-C₁₈-Alkoxycarbonyle, Hydroxycarbonyl, C₁-C₁₈-Alkoxy-C₂ -C₁₈-Alkenyle, C₆-C₁₈-Aryloxy-C₁-C₁₈-Alkyle, C₆-C₁₈-Aryloxy-C₂-C₁₈-Alkenyle, C₃-C₈-Cycloalkyle, C₃-C₈-Cycloalkyl-C₁-C₁₈-Alkyle, C₃-C₈-Cycloalkyl-C₂-C₁₈-Alkenyle
oder R¹ und R² zusammen mit dem Kohlenstoffatom, an das sie gebunden sind, ein asymmetrisches, substituiertes, unsubstituiertes oder Heteroatom-enthaltendes Cycloalkyliden bilden und
soweit es sich bei den Resten R¹ und R² um substituierte Reste handelt, diese Substituenten aus der Gruppe der Alkyl-, Alkenyl-, Alkinyl-, Aryl-, Heteroaryl-, Hydroxy, Alkoxy-, Acyloxy, Silyloxy, Carboxylat-, Alkoxycarbonyl-, Amino-, Nitro- oder Halogenreste ausgewählt werden.

3. Verfahren nach einem oder mehreren der Ansprüche 1 bis 2, **dadurch gekennzeichnet, dass** das Nucleophil die allgemeine Formel NuH besitzt, wobei Nu OR⁵, SR⁵ oder NR⁶R⁷ bedeuten kann und
R⁵ ausgewählt wird aus der Gruppe enthaltend Wasserstoff, substituiertes oder unsubstituiertes C₁-C₁₈-Alkyl, C₂-C₁₈-Alkenyl, C₂-C₁₈-Alkinyl, C₆-C₁₈-Aryl-C₁-C₁₈-Alkyl, C₃-C₁₈-Heteroaryl-C₁-C₁₈-Alkyl, C₆-C₁₈-Aryl-C₂-C₁₈-Alkenyl, C₃-C₁₈-Heteroaryl-C₂-C₁₈-Alkenyl und
R⁶ und R⁷ gleich oder verschieden sein können und unabhängig voneinander ausgewählt werden aus der Gruppe enthaltend Wasserstoff, substituiertes oder unsubstituiertes C₁-C₁₈-Alkyl, C₂-C₁₈-Alkenyl, C₂-C₁₈-Alkinyl-C₆-C₁₈-Aryl, C₃-C₁₈-Heteroaryl, C₆-C₁₈-Aryl-C₁-C₁₈-Alkyl, C₃-C₁₈-Heteroaryl-C₁-C₁₈-Alkyl, C₆-C₁₈-Aryl-C₂-C₁₈-Alkenyl, C₃-C₁₈-Heteroaryl-C₂-C₁₈-Alkenyl und
soweit es sich bei den Resten R⁵, R⁶ und R⁷ um substituierte Reste handelt, werden diese Substituenten ausgewählt aus der Gruppe der gegebenenfalls wiederum substituierten Alkyl-, Alkenyl-, Alkinyl-, Aryl-, Heteroaryl-, Hydroxy-, Alkoxy-, Silyloxy-, Acyloxy-, Carboxylat-, Alkoxycarbonyl-, Amino-, Nitro- oder Halogenreste.

4. Verfahren nach einem oder mehreren der Ansprüche 1 bis 3, **dadurch gekennzeichnet, dass** die Lipase eine Candida antarctica Lipase Typ B (CAL-B) ist.

5. Verfahren nach einem oder mehreren der Ansprüche 1 bis 4, **dadurch gekennzeichnet, dass** die Lipase in freier oder immobilisierter Form eingesetzt wird.

6. Verfahren nach einem oder mehreren der Ansprüche 1 bis 5, **dadurch gekennzeichnet, dass** die Lipase in einem Enzym-Substrat-Molverhältnis von 1 : 1.000 bis 1 : 50.000.000 zugesetzt wird.

7. Verfahren nach einem oder mehreren der Ansprüche 1 bis 6, **dadurch gekennzeichnet, dass** das Nucleophil direkt als Lösungsmittel oder gegebenenfalls in Mischungen mit einem oder mehreren anderen aprotischen oder protogenen Lösungsmitteln als Cosolvens eingesetzt wird.

8. Verfahren nach einem oder mehreren der Ansprüche 1 bis 7, **dadurch gekennzeichnet, dass** das Cosolvens ausgewählt wird aus der Gruppe enthaltend aliphatische Kohlenwasserstoffe, aromatische Kohlenwasserstoffe, halogenierte Kohlenwasserstoffe, Ether, Alkohole, Ester oder Acetonitril oder Mischungen der genannten Verbindungen.

9. Verfahren nach einem oder mehreren der Ansprüche 1 bis 8, **dadurch gekennzeichnet, dass** das Nucleophil Wasser oder ein verzweigter oder unverzweigter C₁-C₆-Alkohol ist.

## Claims

1. Process for the enantioselective preparation of secondary alcohols by enantioselective enzymatic solvolysis of a racemic or enantiomerically enriched mixture of the acetoacetic esters of the chiral secondary alcohols in the presence of a nucleophile, **characterized in that** a lipase capable of the solvolytic cleavage of an ester group is used.

2. Process according to Claim 1, **characterized in that** a racemic or enantiomerically enriched mixture comprising acetoacetic esters of the general formula (1) and (2) 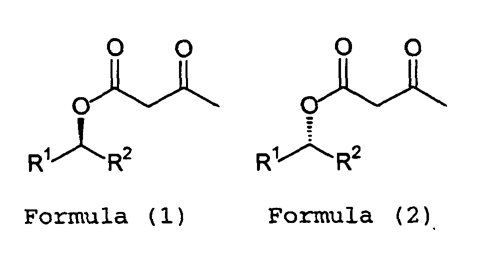 where R¹ is different from R² and
the radicals R¹ and R² are selected independently from the group consisting of substituted or unsubstituted C₆-C₁₈-aryls, C₃-C₁₈-heteroaryls, C₁-C₁₈-alkyls, C₂-C₁₈-alkenyls, C₂-C₁₈-alkynyls, C₆-C₁₈-aryl-C₁-C₁₈-alkyls, C₃-C₁₈-heteroaryl-C₁-C₁₈-alkyls, C₆-C₁₈-aryl-C₁-C₁₈-alkenyls, C₃-C₁₈-heteroaryl-C₁-C₁₈-alkenyls, C₁-C₁₈-alkoxy-C₁-C₁₈-alkyls, C₁-C₁₈-alkoxycarbonyls, hydroxycarbonyl, C₁-C₁₈-alkoxy-C₂-C₁₈-alkenyls, C₆-C₁₈ -aryloxy-C₁-C₁₈-alkyls, C₆-C₁₈-aryloxy-C₂-C₁₈-alkenyls, C₃-C₈-cycloalkyls, C₁-C₈-cycloalkyl-C₁-C₁₈-alkyls, C₁-C₈-cycloalkyl-C₂-C₁₈-alkenyls,
or R¹ and R² together with the carbon atom to which they are bound form an asymmetric, substituted, unsubstituted or heteroatom-containing cycloalkylidene and, if the radicals **R**^{**1**} and **R**^{**2**} are substituted radicals, the substituents are selected from the group consisting of alkyl, alkenyl, alkynyl, aryl, heteroaryl, hydroxy, alkoxy, acyloxy, silyloxy, carboxylate, alkoxycarbonyl, amino, nitro and halogen radicals,
is used.

3. Process according to one or more of Claims 1 and 2, **characterized in that** the nucleophile has the general formula NuH, where Nu can be OR⁵, SR⁵, or NR⁶R⁷ and
R⁵ is selected from the group consisting of hydrogen, substituted or unsubstituted C₁-C₁₈-alkyl, C₂-C₁₈-alkenyl, C₂-C₁₈-alkynyl, C₆-C₁₈-aryl-C₁-C₁₈-alkyl, C₃-C₁₈-heteroaryl-C₁-C₁₈-alkyl, C₆-C₁₈-aryl-C₂-C₁₈-alkenyl, C₁-C₁₈-heteroaryl-C₂-C₁₈-alkenyl and
R⁶ and R⁷ may be identical or different and are selected independently from the group consisting of hydrogen, substituted or unsubstituted C₁-C₁₈-alkyl, C₂-C₁₈-alkenyl, C₂-C₁₈-alkynyl C₆-C₁₈-aryl, C₃-C₁₈-heteroaryl, C₆-C₁₈-aryl-C₁-C₁₈-alkyl, C₃-C₁₈-heteroaryl-C₁-C₁₈-alkyl, C₆-C₁₈-aryl-C₂-C₁₈-alkenyl, C₃-C₁₈-heteroaryl-C₂-C₁₈-alkenyl, and
if the radicals R⁵, R⁶ and R⁷ are substituted radicals, the substituents are selected from the group consisting of unsubstituted or in turn substituted alkyl, alkenyl, alkynyl, aryl, heteroaryl, hydroxy, alkoxy, silyloxy, acyloxy, carboxylate, alkoxycarbonyl, amino, nitro and halogen radicals.

4. Process according to one or more of Claims 1 to 3, **characterized in that** the lipase is a Candida antarctica lipase type B (CAL-B).

5. Process according to one or more of Claims 1 to 4, **characterized in that** the lipase is used in free or immobilized form.

6. Process according to one or more of Claims 1 to 5, **characterized in that** the lipase is added in an enzyme/substrate molar ratio of from 1 : 1000 to 1 : 50 000 000.

7. Process according to one or more of Claims 1 to 6, **characterized in that** the nucleophile is used directly as solvent or, if desired, in mixtures with one or more other aprotic or protogenic solvents as cosolvent.

8. Process according to one or more of Claims 1 to 7, **characterized in that** the cosolvent is selected from the group consisting of aliphatic hydrocarbons, aromatic hydrocarbons, halogenated hydrocarbons, ethers, alcohols, esters and acetonitrile and mixtures of the compounds mentioned.

9. Process according to one or more of Claims 1 to 8, **characterized in that** the nucleophile is water or a branched or unbranched C₁-C₆-alcohol.

## Revendications

1. Procédé pour la préparation énantio-sélective d'alcools secondaires par solvolyse enzymatique énantio-sélective d'un mélange racémique ou enrichi en énantiomères des esters de l'acide acétoacétique des alcools secondaires chiraux en présence d'un nucléophile, **caractérisé en ce qu'**on utilise une lipase apte au clivage par solvolyse d'un groupement ester.

2. Procédé selon la revendication 1, **caractérisé en ce qu'**on utilise un mélange racémique ou enrichi en énantiomères contenant des esters acétoacétiques de formule générale (1) et (2) 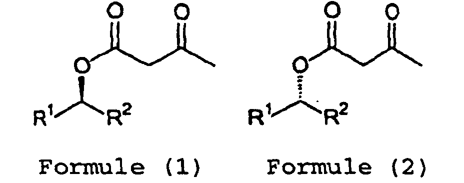 R¹ étant différent de R² et
les radicaux R¹ et R² étant choisis indépendamment l'un de l'autre du groupe contenant des groupements aryle en C₆ à C₁₈, hétéroaryle en C₃ à C₁₈, alkyle en C₁ à C₁₈, alcényle en C₂ à C₁₈, alcynyle en C₂ à C₁₈, (aryle en C₆ à C₁₈) (alkyle en C₁ à C₁₈), (hétéroaryle en C₃ à C₁₈) (alkyle en C₁ à C₁₈), (aryle en C₆ à C₁₈) (alcényle en C₁ à C₁₈), (hétéroaryle en C₃ à C₁₈) (alcényle en C₁ à C₁₈), (alcoxy en C₁ à C₁₈) (alkyle en C₁ à C₁₈), (alcoxy en C₁ à C₁₈) carbonyle, hydroxycarbonyle, (alcoxy en C₁ à C₁₈) (alcényle en C₂ à C₁₈), (aryloxy en C₆ à C₁₈) (alkyle en C₁ à C₁₈), (aryloxy en C₆ à C₁₈) (alcényle en C₂ à C₁₈), cycloalkyle en C₃ à C₈, (cycloalkyle en C₃ à C₈) (alkyle en C₁ à C₁₈), (cycloalkyle en C₃ à C₈) (alcényle en C₂ à C₁₈), substitués ou non substitués
ou R¹ et R² forment ensemble avec l'atome de carbone auquel ils sont liés, un cycloalkylidène asymétrique, substitué ou non substitué ou contenant un hétéroatome et, pour autant qu'il s'agisse pour les radicaux R¹ et R² de radicaux substitués, les substituants sont choisis
dans le groupe constitué par les radicaux alkyle, alcényle, alcynyle, aryle, hétéroaryle, hydroxy, alcoxy, acyloxy, silyloxy, carboxylate, alcoxycarbonyle, amino, nitro ou halogène.

3. Procédé selon l'une ou plusieurs des revendications 1 à 2, **caractérisé en ce que** le nucléophile présente la formule générale NuH, Nu pouvant signifier OR⁵, SR⁵ ou NR⁶R⁷ et
R⁵ est choisi dans le groupe contenant hydrogène, alkyle en C₁ à C₁₈, alcényle en C₂ à C₁₈, alcynyle en C₂ à C₁₈, (aryle en C₆ à C₁₈) (alkyle en C₁ à C₁₈), (hétéroaryle en C₃ à C₁₈) (alkyle en C₁ à C₁₈), (aryle en C₆ à C₁₈) (alcényle en C₂ à C₁₈), (hétéroaryle en C₃ à C₁₈) (alcényle en C₂ à C₁₈), substitués ou non substitués et
R⁶ et R⁷ peuvent être identiques ou différents et sont choisis, indépendamment l'un de l'autre du groupe contenant hydrogène, alkyle en C₁ à C₁₈, alcényle en C₂ à C₁₈, (alcynyle en C₂ à C₁₈) (aryle en C₆ à C₁₈), hétéroaryle en C₃ à C₁₈, (aryle en C₆ à C₁₈) (alkyle en C₁ à C₁₈), (hétéroaryle en C₃ à C₁₈) (alkyle en C₁ à C₁₈), (aryle en C₆ à C₁₈) (alcényle en C₂ à C₁₈), (hétéroaryle en C₃ à C₁₈) (alcényle en C₂ à C₁₈), substitués ou non substitués et
pour autant qu'il s'agisse, pour les radicaux R⁵, R⁶ et R⁷, de radicaux substitués, ces substituants sont choisis dans le groupe des radicaux, le cas échéant à nouveau substitués, alkyle, alcényle, alcynyle, aryle, hétéroaryle, hydroxy, alcoxy, silyloxy, acyloxy, carboxylate, alcoxycarbonyle, amino, nitro ou halogène.

4. Procédé selon l'une ou plusieurs des revendications 1 à 3, **caractérisé en ce que** la lipase est une lipase de Candida antarctica de type B (CAL-B).

5. Procédé selon l'une ou plusieurs des revendications 1 à 4, **caractérisé en ce que** la lipase est utilisée sous forme libre ou immobilisée.

6. Procédé selon l'une ou plusieurs des revendications 1 à 5, **caractérisé en ce que** la lipase est ajoutée en un rapport molaire enzyme-substrat de 1 : 1000 à 1 : 50 000 000.

7. Procédé selon l'une ou plusieurs des revendications 1 à 6, **caractérisé en ce que** le nucléophile est utilisé directement en tant que solvant ou le cas échéant dans des mélanges avec un ou plusieurs autres solvants àprotiques ou protogènes comme cosolvant.

8. Procédé selon l'une ou plusieurs des revendications 1 à 7, **caractérisé en ce que** le cosolvant est choisi dans le groupe contenant les hydrocarbures aliphatiques, les hydrocarbures aromatiques, les hydrocarbures halogénés, les éthers, les alcools, les esters ou l'acétonitrile ou des mélanges des composés mentionnés.

9. Procédé selon l'une ou plusieurs des revendications 1 à 8, **caractérisé en ce que** le nucléophile est l'eau ou un alcool en C₁ à C₆ ramifié ou non ramifié.
